# FASCICULE DE BREVET EUROPEEN

(11) **EP 3 986 503 B1**
(45) Date de publication et mention de la délivrance du brevet: **19.07.2023**
(21) Numéro de dépôt: 20743187.5
(22) Date de dépôt: 27.05.2020
(51) Int. Cl.: A61M 3/02, A61F 5/442, A61F 2/04

(54) **KIT POUR LE LAVEMENT D'UNE ANASTOMOSE INTESTINALE IN SITU**
KIT ZUM WASCHEN EINER DARMANASTOMOSE VOR ORT
KIT FOR WASHING AN INTESTINAL ANASTOMOSIS IN SITU

(30) Priorité: 24.06.2019 FR 1906808
(43) Date de publication de la demande: 27.04.2022
(73) Titulaire: SafeHeal SAS, 75002 Paris (FR)
(72) Inventeur: LEVEAU-MOLLIER, Séverine, 91570 BIEVRES (FR)
(74) Mandataire: Vossius & Partner Patentanwälte Rechtsanwälte mbB
(86) Numéro de dépôt international: PCT/FR2020/050889
(87) Numéro de publication internationale: WO 2020/260784

(56) Documents cités:
- CN-A- 108 261 577
- FR-A1- 2 941 858

## Description

### Domaine Technique

La présente invention est relative au domaine du soin d'une anastomose et concerne en particulier un kit pour le lavement d'une anastomose intestinale in situ.

### Technique antérieure

Les anastomoses correspondent à la connexion entre deux structures, organes ou espaces, par couture ou par agrafes. Une anastomose intestinale par exemple, correspond à l'union de deux extrémités d'un segment de l'intestin pouvant intervenir notamment après résection d'un segment de l'intestin. On peut notamment faire référence aux anastomoses réalisées dans le cadre de la résection d'une partie du colon dans les cas de cancer colorectal où une anastomose dite basse est pratiquée.

La désunion d'une anastomose (fistule) est une complication grave avec une mortalité avoisinant les 20%, en particulier dans les cas d'anastomoses intestinales notamment colorectales ou colo anales. Les fistules anastomotiques correspondent à une communication anormale de deux cavités ayant pour conséquence le déversement du contenu d'une cavité dans l'autre cavité.

Les fistules anastomotiques sont généralement causées par une mauvaise cicatrisation de la suture de l'anastomose et se produisent en général une semaine après l'opération. Les symptômes peuvent être de la fièvre, l'interruption du transit intestinal, des douleurs intestinales plus ou moins violentes ou encore un choc septique susceptible de mettre la vie du patient en danger. Dans d'autres cas le patient peut ne présenter aucun symptôme.

Différents traitements peuvent être envisagés en fonction de la gravité de la fistule anastomotique tel qu'une antibiothérapie pour les cas les moins graves ou encore une endoscopie ou une nouvelle chirurgie présentant des risques encore plus élevés que la première pour les cas les plus graves.

Etant donné les conséquences sévères pouvant être associées aux anastomoses notamment intestinales, il existe donc un besoin de traiter et / ou prévenir les conséquences thérapeutiques liées à la réalisation d'une anastomose, telles que les difficultés de cicatrisation ou encore les infections bactériennes, et in fine les fistules anastomotiques. Afin de prévenir l'apparition de fistules anastomotiques et de façon plus générale les conséquences thérapeutiques liées à la réalisation d'une anastomose notamment intestinale, il serait avantageux de pouvoir disposer d'une solution technique permettant de distribuer directement et de façon peu invasive au niveau de l'anastomose une composition pouvant comprendre un ou plusieurs composé(s) actif(s).

Pour ce faire, il pourrait être avantageux de procéder à des lavements réguliers, répétés de l'anastomose pour faciliter sa guérison.

Les solutions disponibles à ce jour permettant de réaliser le lavement d'une anastomose intestinale sont généralement mises en oeuvre de façon classique par voie anale et nécessitent l'introduction du dispositif de lavement lors de chaque lavement et donc un passage répété au travers de l'anastomose ou l'application d'une pression de liquide dans la zone de l'anastomose par exemple à l'aide d'un bock à lavement, d'une poire à canule ou d'une poire simple.

Les lavements peuvent également être réalisés par voie percutanée. Par exemple par lavement antégrade selon la méthode de Malone qui nécessite une intervention chirurgicale pour la réalisation d'une stomie permettant de créer un accès au liquide de lavement, ou encore par caecostomie percutanée réalisée par voie endoscopique (CPE) qui nécessite la création d'un orifice de caecostomie entre la première partie du côlon et la peau, pour l'introduction du liquide de lavement. Cependant, ces lavements percutanés sont des techniques invasives présentant des risques médicaux pour le patient tels que des infections et des inflammations. Les différentes solutions existant pour le lavement d'une anastomose intestinale sont donc invasives puisqu'elles nécessitent soit l'introduction répétée d'un dispositif de lavement qui risque d'endommager l'anastomose et d'altérer sa cicatrisation, favorisant ainsi l'apparition de fistules anastomotiques ; soit la création d'un orifice sur l'abdomen du patient pour permettre l'accès au liquide sans introduction anale. C'est pourquoi les procédures de lavement d'anastomose intestinale, notamment de façon répétée, ne sont pratiquement jamais utilisées dans le cadre du traitement et / ou de la prévention des conséquences thérapeutiques liées à la réalisation d'une anastomose intestinale. FR 2 941 858 A1 divulgue un dispositif de protection d'anastomoses. CN 108 261 577 A divulgue un dispositif d'irrigation d'anastomoses.

La présente invention a donc pour objectif de proposer une solution technique permettant de traiter et / ou prévenir les complications thérapeutiques liées à la réalisation d'une anastomose intestinale et notamment de prévenir les fistules anastomotiques, qui soit peu invasive, permettant de réaliser des lavements répétés in situ ne nécessitant pas de multiples réintroduction in vivo du dispositif de lavement, efficace, et adaptable au contexte d'utilisation.

La présente invention cible les complications thérapeutiques liées à la réalisation d'une anastomose de façon générale telles que les difficultés de cicatrisation, les infections bactériennes, les douleurs au niveau de l'anastomose ou encore les fistules anastomotiques. Toutes ces complications thérapeutiques méritent de pouvoir être traitées et / ou prévenues de façon efficace et peu invasive.

A cet effet, la présente invention propose un kit pour le lavement d'une anastomose intestinale in situ, permettant de réaliser des lavements répétés et ne nécessitant pas d'être réintroduit lors de chaque lavement.

### Exposé de l'invention

Un objet de l'invention concerne un kit comprenant un dispositif (1) pour le lavement d'une anastomose intestinale (2) in situ et un dispositif de protection d'anastomose comprenant une gaine externe souple (9) liée à un stent (7) situé en aval de la gaine, ledit stent étant destiné à être ancré en amont de l'anastomose, le dispositif (1) pour le lavement comprenant un élément tubulaire perforé de forme annulaire (3), apte à distribuer une composition de lavement en amont de l'anastomose, un tube d'alimentation (4) dont une extrémité (4a) débouche dans l'élément tubulaire et une extrémité (4b) s'ouvre vers des moyens d'injection (5) destinés à apporter la composition dans ledit élément tubulaire, des moyens de maintien (6) de l'élément tubulaire en amont de l'anastomose.

### Brève description des dessins

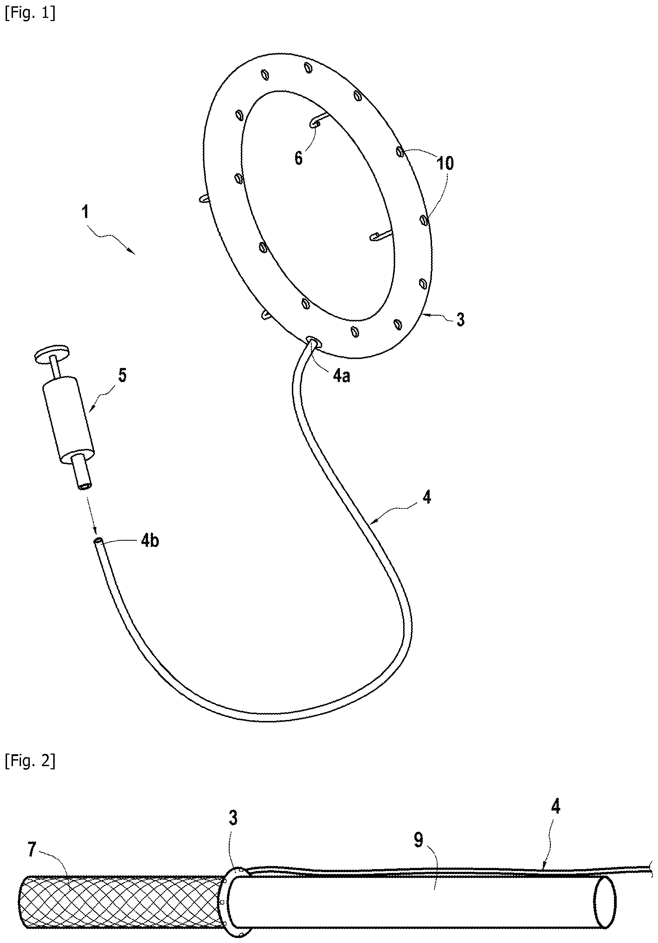

[Fig. 1] La figure 1 représente le dispositif de lavement (1) d'une anastomose selon l'invention comprenant un élément tubulaire perforé de forme annulaire (3), un tube d'alimentation (4) dont une extrémité (4a) débouche dans l'élément tubulaire et une extrémité (4b) s'ouvre vers des moyens d'injection (5) destinés à apporter la composition dans ledit élément tubulaire et des moyens de maintien (6) de l'élément tubulaire en amont de l'anastomose.
[Fig. 2] La figure 2 représente schématiquement le dispositif de lavement (1) selon la présente invention couplé à un dispositif de protection d'anastomose comprenant un élément d'ancrage (7) et une gaine externe (9) située à l'extrémité avale de l'élément d'ancrage (7) dans lequel l'élément tubulaire (3) du dispositif de lavement est fixé à la partie avale de l'élément d'ancrage par des moyens de maintien (6).

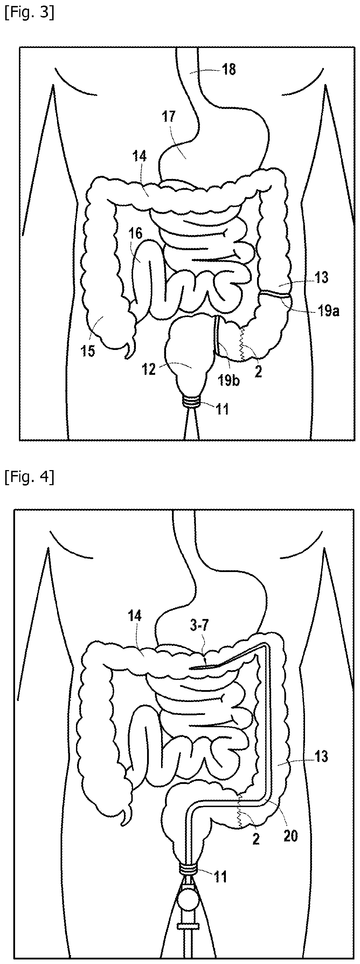

[Fig. 3] La figure 3 représente une vue schématique du système digestif avec une anastomose (2) reliant les deux extrémités du segment d'intestin prélevé (19a et 19b) dans lequel on retrouve le rectum (11), le canal anal (12), le côlon gauche (13), le côlon transverse (14), le côlon droit (15), le petit intestin (16), l'estomac (17) et l'oesophage (18).
[Fig. 4] La figure 4 représente une vue schématique du dispositif de lavement (1) selon l'invention couplé au dispositif de protection d'anastomose acheminés vers la position en amont de l'anastomose à travers un tube introducteur (20), l'élément tubulaire (3) et l'élément d'ancrage (7) étant en position radiale rétractée au sortir de l'introducteur, la gaine externe (9) et le tube d'alimentation (4) étant encore logés à l'intérieur du tube introducteur.

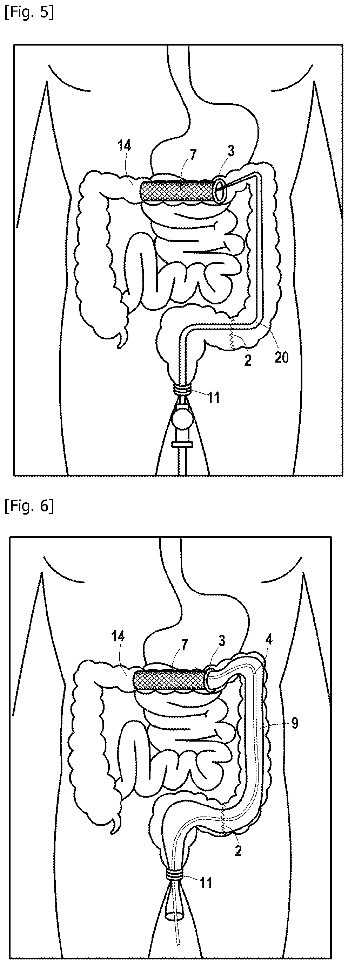

[Fig. 5] La figure 5 représente une vue schématique du dispositif de lavement (1) selon l'invention couplé au dispositif de protection d'anastomose en partie positionnés en amont de l'anastomose, l'élément tubulaire (3) et l'élément d'ancrage (7) auquel il est fixé étant en position d'expansion radiale maximale, maintenus contre la paroi de l'intestin.
[Fig. 6] La figure 6 représente schématiquement le dispositif de lavement (1) selon l'invention couplé au dispositif de protection d'anastomose avec le tube d'alimentation (4) du dispositif de lavement et la gaine externe (9) du dispositif de protection d'anastomose déployés en aval de l'élément d'ancrage (7) du dispositif de protection d'anastomose.

### Description des modes de réalisation

Les Demandeurs ont mis au point un dispositif pour le lavement d'une anastomose intestinale in situ. Ce dispositif est particulièrement intéressant pour pouvoir distribuer directement, de façon répétée et peu invasive une composition dite de lavement en amont d'une anastomose, ladite composition pouvant comprendre un ou plusieurs composé(s) actif(s), afin de traiter et / ou prévenir les complications thérapeutiques liées à la réalisation d'une anastomose.

Un premier objet de l'invention concerne un dispositif (1) pour le lavement d'une anastomose intestinale (2) in situ, ledit dispositif comprenant un élément tubulaire perforé de forme annulaire (3), apte à distribuer une composition de lavement en amont de l'anastomose, un tube d'alimentation (4) dont une extrémité (4a) débouche dans l'élément tubulaire et une extrémité (4b) s'ouvre vers des moyens d'injection (5) destinés à apporter la composition dans ledit élément tubulaire, des moyens de maintien (6) de l'élément tubulaire en amont de l'anastomose.

En particulier, le dispositif selon la présente invention concerne un dispositif pour le lavement d'une anastomose intestinale in situ, ledit dispositif étant apte à être maintenu in situ pendant la période pendant laquelle les lavements de l'anastomose sont réalisés. Dans le cadre de la présente invention, la disposition, le maintien in situ du dispositif signifie également in vivo puisque l'objectif de l'invention est le lavement d'une anastomose intestinale.

Cette installation et le maintien *in situ* du dispositif selon la présente invention pendant la période de lavement de l'anastomose, permet avantageusement de réaliser le lavement d'une anastomose de façon répétée et peu invasive, puisqu'il permet d'éviter le passage par l'anastomose lors de chaque lavement risquant d'endommager et d'altérer ladite anastomose et en particulier sa suture.

En effet cette installation ne nécessite pas de multiples passages du dispositif à travers l'anastomose fraîchement réalisée. Le dispositif selon la présente invention peut en effet être disposé avant la réalisation de l'anastomose et utiliser l'excision du segment intestinal pathologique et l'ouverture de la lumière dudit segment requise pour la mise en place du dispositif selon l'invention. Il peut également être disposé après la réalisation de l'anastomose via l'orifice anal et ne nécessiter qu'un seul passage au travers de l'anastomose fraîchement réalisée. Dans les deux cas, le dispositif sera maintenu in situ pendant toute la période pendant laquelle des lavements répétés seront réalisés.

La distribution directe d'une composition de lavement en amont de l'anastomose, permet de présenter une efficacité locale améliorée du lavement mis en oeuvre par le présent dispositif et en particulier de la composition de lavement distribuée pour le traitement et / ou la prévention des complications thérapeutiques liées à la réalisation d'une anastomose. Plus particulièrement, le dispositif selon la présente invention est destiné au lavement d'une anastomose intestinale, c'est-à-dire d'une anastomose réalisée au niveau des intestins et de préférence d'une anastomose intestinale dite basse telle qu'une anastomose sur le côlon, le rectum ou le canal anal par exemple une anastomose colorectale ou colo-anale correspondant respectivement à un abouchement entre le colon et le rectum ou entre le colon et l'anus.

Par « lavement » dans le cadre de la présente invention, on fait référence à la distribution d'une composition de lavement sur l'anastomose et en particulier en amont de l'anastomose et en direction de ladite anastomose. Le lavement dans le cadre de l'invention peut faire intervenir différents types de compositions de lavement en fonction du contexte d'utilisation du dispositif.

On entend par composition de lavement dans le cadre de l'invention, toute composition, de préférence liquide, destinée à être distribuée en amont de l'anastomose et pouvant comprendre un ou plusieurs composé(s) actif(s). La composition de lavement étant choisie en fonction du contexte d'utilisation du dispositif.

On entend par « contexte d'utilisation du dispositif » au sens de l'invention, le ou les objectifs de la mise en oeuvre du dispositif de lavement selon la présente invention, et plus particulièrement le ou les objectifs, le ou les indications visées par la distribution d'une composition de lavement en amont d'une anastomose lors de la mise en oeuvre dudit dispositif. Le contexte d'utilisation du dispositif, c'est-à-dire le ou les objectifs de la mise en oeuvre du dispositif peuvent être divers, tels que le nettoiement de l'anastomose, l'aide à la cicatrisation et de façon plus générale, le traitement et /ou la prévention des complications thérapeutiques liées à la réalisation d'une anastomose.

On entend par « complication(s) thérapeutique(s) » au sens de la présente invention, les troubles ou désordres physiques et / ou physiologiques pouvant faire suite, être liés à la réalisation d'une anastomose et dont l'occurrence peut être fréquente et normale suite à cette intervention ou d'ordre pathologique. Parmi ces troubles ou désordres on peut faire référence à une infection bactérienne et / ou virale, à une douleur au niveau de l'anastomose, à une difficulté de cicatrisation de l'anastomose ou encore aux fistules anastomotiques.

Par « in vivo », on fait référence ici à l'intérieur d'un être vivant, d'un sujet, en particulier aux intestins et encore plus particulièrement au segment intestinal situé en amont de l'anastomose.

Par « in situ », on fait référence dans la présente invention à la position in vivo dans laquelle le dispositif est installé et maintenu pendant la période pendant laquelle les lavements sont réalisés, plus précisément on fait référence au segment intestinal situé en amont de l'anastomose dans lequel le dispositif est installé et maintenu pendant la période pendant laquelle les lavements sont réalisés. Le dispositif selon la présente invention est donc installé et maintenu in vivo et in situ.

Par « protection de l'anastomose », on entend ici la protection de l'anastomose lors de la reprise du transit intestinal, intervenant en moyenne 3 à 5 jours après la réalisation de l'anastomose, cette période correspondant à la phase de paralysie intestinale postopératoire.

Par « en amont de l'anastomose » on fait référence à la position située avant le site de l'anastomose en considérant le sens du transit intestinal, en particulier à la position située en avant de l'anastomose et dans laquelle le dispositif selon l'invention est destiné à être installé. La position dite en amont de l'anastomose est comprise entre 1m et 1 cm, de préférence entre 50 cm et 15 cm, de préférence encore entre 30 et 15 cm en amont de l'anastomose.

Les moyens de maintien (6) de l'élément tubulaire dans le cadre de la présente invention comprennent des éléments permettant l'accroche directe ou indirecte de l'élément tubulaire, en amont de l'anastomose et plus précisément sur la paroi interne du segment intestinal situé en amont de l'anastomose.

En particulier, les moyens de maintien (6) du dispositif selon la présente invention sont disposés sur l'élément tubulaire.

Les moyens de maintien (6) peuvent être des éléments d'accroche tels que des boucles, des pattes, des crochets, utilisés seuls ou en combinaison.

De préférence, les moyens de maintien (6) comprennent des éléments d'accroche indirecte de l'élément tubulaire perforé de forme annulaire du dispositif selon la présente invention sur la paroi interne du segment intestinal situé en amont de l'anastomose (2).

De préférence encore, les moyens de maintien du dispositif selon la présente invention sont aptes à se fixer sur un élément d'ancrage temporaire (7) tel qu'un stent.

En particulier, les moyens de maintien (6) du dispositif selon la présente invention sont aptes à se fixer sur un stent (7).

De préférence, le stent est compris dans un dispositif de protection d'anastomose intestinale.

De préférence encore, les moyens de maintien (6) selon la présente invention sont aptes à se fixer sur le stent (7) d'un dispositif de protection d'anastomose intestinale destiné et apte à être inséré et maintenu in situ sur la paroi interne du segment anatomique intestinal situé en amont de l'anastomose (2).

Plus particulièrement, les moyens de maintien (6) du dispositif selon la présente invention sont aptes à se fixer sur le stent (7) d'un dispositif de protection d'anastomose intestinale comprenant une gaine externe souple (9) liée audit stent situé en aval de la gaine, ledit stent étant destiné à être ancré en amont de l'anastomose. Dans le cadre de la présente invention, les moyens de maintien (6) sont donc aptes à fixer l'élément tubulaire (3) du dispositif pour le lavement, sur le stent (7) du dispositif de protection d'anastomose.

Le dispositif selon la présente invention est dit couplé ou jumelé au dispositif de protection d'une anastomose lorsque les moyens de maintien (6) du dispositif de lavement sont fixés sur le stent du dispositif de protection d'anastomose.

Selon un premier mode de réalisation, les moyens de maintien fixent l'élément tubulaire du dispositif selon la présente invention sur la partie interne du stent du dispositif de protection d'anastomose.

Selon un autre mode de réalisation, les moyens de maintien (6) fixent l'élément tubulaire du dispositif selon la présente invention sur la partie externe du stent (7) du dispositif de protection d'anastomose.

Selon un mode de réalisation préféré, les moyens de maintien (6) fixent l'élément tubulaire (3) du dispositif selon la présente invention sur l'extrémité avale du stent (7) du dispositif de protection d'anastomose et de préférence sur la partie externe dudit stent. De préférence dans ce mode de réalisation, les moyens de maintien (6) sont situés à proximité, vers, le diamètre interne de l'élément tubulaire.

Dans chacun de ces modes de réalisation, les moyens de maintien (6) fixent l'élément tubulaire (3) du dispositif selon la présente invention à l'extérieur de la gaine externe (9) du dispositif de protection d'anastomose afin de permettre à la composition de lavement d'être distribuée en amont de l'anastomose.

Le dispositif de protection d'anastomose sur lequel les moyens de maintien sont aptes à se fixer, est un dispositif de protection d'une anastomose intestinale tel que décrit dans les documents brevets suivants FR 2 941 858, EP 2 395 942, WO2013/014353 ou encore dans la demande de brevet WO2019/077218.

Le dispositif de protection de l'anastomose intestinale sur lequel les moyens de maintien (6) sont aptes à se fixer, c'est-à-dire auquel est couplé le dispositif selon la présente invention, est composé d'une gaine externe souple (9) solidarisée à un stent (7) en aval de celui-ci, le dit stent étant destiné à être ancré en amont de l'anastomose. Le stent est l'élément d'ancrage temporaire aux parois de l'intestin, qui maintient la gaine en place et la gaine sert à dériver les matières fécales vers l'orifice anal sans contact avec la paroi intestinale au niveau de l'anastomose et ainsi protéger l'anastomose.

Plus précisément, le dispositif de protection de l'anastomose sur lequel les moyens de maintien (6) sont aptes à se fixer, comprend un élément d'ancrage temporaire (7), comprenant au moins un premier élément longitudinal creux semi-rigide du type stent, définissant une paroi de révolution autour d'un axe longitudinal comprenant une partie courante multi-perforée sensiblement cylindrique à section sensiblement circulaire dite première paroi, ledit premier élément longitudinal creux étant réalisé en un matériau lui conférant des propriétés d'élasticité radiale de sorte qu'il peut être comprimé radialement en position rétractée et adopter une dite position d'expansion radiale maximale après relâchement de la compression radiale telle que ladite première paroi multi-perforée présente un premier diamètre externe que l'on peut faire varier de façon contrôlée entre un premier diamètre externe minimal en dite position radiale rétractée de ladite première paroi et un premier diamètre externe maximal en dite position d'expansion radiale maximale de ladite première paroi, et une gaine externe souple (9) fixée au dit stent et s'étendant depuis son extrémité avale. Ces propriétés d'élasticité de l'élément d'ancrage du dispositif de protection d'anastomose, permettent l'installation et le maintien in situ en amont de l'anastomose, de ce dispositif et par conséquent du dispositif selon la présente invention qui y est couplé et plus particulièrement de l'élément tubulaire perforé (3) du dispositif selon la présente invention qui présente également des propriétés d'élasticité.

Le dispositif de protection d'anastomose peut également sur au moins une partie, de préférence toute la longueur, de la surface interne de l'élément d'ancrage être doublé d'une couche étanche indépendante formant une gaine interne, seules les extrémités longitudinales de ladite gaine interne étant fixées de manière étanche à l'élément d'ancrage à l'aide de moyens de fixation étanches, tels qu'un joint annulaire de colle élastomère, à chaque extrémité longitudinale de la gaine interne afin de délimiter une chambre dénommée chambre de dépression entre ladite gaine interne et la surface interne de l'élément d'ancrage, l'élément d'ancrage étant couplé à un tube d'injection-aspiration souple ou semi-rigide s'étendant à l'extérieur dudit élément d'ancrage, une extrémité ouverte dudit tube d'injection-aspiration débouchant dans la chambre de dépression. Le tube d'injection-aspiration est connecté, de préférence de façon réversible à son extrémité longitudinale libre à un embout de raccordement, lui-même connecté de façon réversible ou apte à être connecté de façon réversible à un dispositif d'injection ou aspiration d'air tel qu'une seringue ou un redon, ledit embout de raccordement comprenant un dispositif d'obturation, de préférence une vanne anti-reflux et un dispositif de témoin de vide apte à indiquer la teneur du vide dans ladite chambre de dépression. Le tube d'aspiration d'air permet de maintenir le dispositif de protection d'anastomose in situ en amont de l'anastomose par aspiration d'air depuis son extrémité libre, depuis l'extérieur du sujet pour attirer par aspiration la paroi intestinale contre la surface externe de l'élément d'ancrage délimitant la chambre et établir le vide dans ladite chambre. La combinaison des propriétés d'élasticité de l'élément d'ancrage du dispositif de protection d'anastomose et du tube d'aspiration d'air dans la chambre de dépression présente dans ledit élément d'ancrage permet d'assurer un maintien plus ferme in situ en amont de l'anastomose, du dispositif de protection d'anastomose et par conséquent du dispositif selon la présente invention qui y est couplé.

Le dispositif selon la présente invention est de préférence couplé au dispositif de protection d'une anastomose avant l'installation in situ du dispositif de protection d'une anastomose, afin de faciliter et de sécuriser l'installation du dispositif selon la présente invention.

Le dispositif selon la présente invention couplé au dispositif de protection d'une anastomose peuvent être installés in situ par un dispositif introducteur tel que décrit dans WO2013/014353.

Plus précisément, le dispositif introducteur utile dans le cadre de la présente invention, peut être constitué de façon connue d'un tube guide semi rigide (20) dit tube introducteur, de type cathéter muni à une de ses extrémités d'une poignée et dont le diamètre interne et la longueur du tube guide permettent d'y maintenir logé l'élément d'ancrage donc de préférence le stent du dispositif de protection d'anastomose sous sa forme rétractée, et la gaine sous forme déployée longitudinalement. Le dispositif introducteur est également apte à maintenir logé l'élément tubulaire perforé de forme annulaire du dispositif selon la présente invention sous forme rétractée dans ledit tube guide, et à maintenir le tube d'alimentation du dispositif sous forme déployée longitudinalement.

Pour une introduction par voie anale notamment après réalisation de l'anastomose intestinale, le dispositif introducteur comprend en outre un instrument introducteur comprenant : - une enveloppe externe tubulaire apte à contenir et maintenir l'élément d'ancrage et l'élément tubulaire comprimés sous forme rétractée au sein de l'extrémité distale de ladite enveloppe, et la gaine externe et le tube d'alimentation sous forme déployée longitudinalement - des moyens pour acheminer l'extrémité distale dudit introducteur depuis l'orifice anal jusqu'en amont de l'anastomose et - des moyens pour dégager l'élément d'ancrage et l'élément tubulaire par rapport à l'enveloppe externe, pouvant consister en un tube butoir comportant une butée à son extrémité distale, en contact le cas échéant avec l'extrémité longitudinale dudit élément d'ancrage avec la gaine externe du dispositif de protection située en aval de l'élément d'ancrage entourant le tube butoir à l'intérieur de ladite enveloppe.

Les moyens pour acheminer l'extrémité distale de l'introducteur depuis l'orifice anal jusqu'en amont de l'anastomose peuvent être un pousseur s'étendant depuis la poignée reliée à l'extrémité du tube guide et comprenant une tige de pousseur et une butée de pousseur à l'extrémité distale de la tige de pousseur. La butée du pousseur est apte à pousser le dispositif de protection couplé au dispositif de lavement selon l'invention à l'extérieur de l'extrémité distale du tube guide pour permettre leur extension radiale et l'ancrage de l'élément d'ancrage, notamment du stent, contre la paroi de l'intestin.

Après retrait de l'introducteur, l'élément d'ancrage tel que le stent et l'élément tubulaire qui y est fixé, s'ouvrent en extension radiale et viennent au contact des parois d'intestin tandis que la gaine, le tube d'alimentation et le tube d'aspiration se déploient dans la lumière de l'intestin depuis le site d'ancrage en amont de l'anastomose jusqu'à l'orifice anal en traversant l'anastomose.

Le dispositif selon la présente invention comprend un tube d'alimentation (4) destiné à apporter la composition de lavement à l'élément tubulaire perforé (3) de forme annulaire. A cet effet, une extrémité (4a) du tube d'alimentation débouche dans l'élément tubulaire et une extrémité (4b) s'ouvre vers des moyens d'injection (5) destinés à apporter la composition dans ledit élément tubulaire.

L'extrémité (4a) du tube d'alimentation débouchant dans l'élément tubulaire (3) est fixée audit élément par des moyens de fixation étanches, de préférence par fusion ou par un joint annulaire de colle élastomère. L'extrémité (4b) s'ouvrant vers des moyens d'injection (5) destinés à apporter la composition dans ledit élément tubulaire peut s'ouvrir directement ou indirectement vers lesdits moyens d'injections. Lorsque l'extrémité (4b) du tube d'alimentation s'ouvre indirectement vers les moyens d'injections (5), ladite extrémité est connectée de façon réversible à une pièce de raccordement, elle-même connectée ou apte à être connectée de façon réversible aux moyens d'injections tels qu'une seringue ou une pompe.

En particulier, l'extrémité (4b) du tube d'alimentation (4) qui s'ouvre vers des moyens d'injection (5) est destinée à être située à l'extérieur du corps.

Plus précisément, l'extrémité (4b) du tube d'alimentation qui s'ouvre vers des moyens d'injection, est située à l'extérieur du corps, c'est-à-dire ex vivo, lorsque l'élément tubulaire (3) est installé en amont de l'anastomose.

Selon un aspect préféré, le tube d'alimentation (4) est un tube souple ou semi-rigide.

Dans le cadre de la présente invention, le tube d'alimentation (4) présente un diamètre interne pouvant être compris entre 1 et 8 mm, de préférence entre 2 et 5 mm et de préférence encore le tube présente un diamètre interne inférieur ou égal à 5 mm. Le tube présente un diamètre externe compris entre 2 et 9 mm, de préférence entre 3 et 6 mm. Un tube d'alimentation présentant un diamètre interne inférieur ou égal à 5 mm, permet avantageusement d'assurer l'apport de la composition de lavement jusqu'à l'élément tubulaire perforé de forme annulaire, notamment de faibles quantités de ladite composition.

Le tube d'alimentation (4) selon la présente invention présente une longueur initiale pouvant être ajustée après l'installation du dispositif et en particulier de l'élément tubulaire in situ, de sorte que le tube soit suffisamment long pour que l'extrémité du tube par laquelle la composition est insérée se trouve à l'extérieur du corps. Selon un aspect préféré, le dispositif selon la présente invention comprend un seul tube d'alimentation.

L'élément tubulaire de forme annulaire (3) du dispositif est apte à distribuer une composition de lavement en amont de l'anastomose, pour ce faire ledit élément est disposé, installé, en amont de l'anastomose. La disposition de l'élément tubulaire perforé en amont de l'anastomose permet à la composition de couler en direction de l'anastomose jusqu'à l'atteindre. De préférence le dispositif selon la présente invention comprend un seul élément tubulaire perforé de forme annulaire (3).

Selon un aspect particulièrement préféré, l'élément tubulaire (3) du dispositif selon la présente invention, est réalisé en matériau lui conférant des propriétés d'élasticité radiale de sorte qu'il peut être comprimé radialement en position rétractée et adopter une position d'expansion radiale maximale après relâchement de la compression radiale. Ces propriétés permettent avantageusement à l'élément tubulaire de pouvoir s'adapter aux variations de diamètre de l'élément d'ancrage du dispositif de protection auquel il est fixé, notamment lors du logement au sein du tube guide du dispositif introducteur. En d'autres termes, les propriétés d'élasticité radiale de l'élément tubulaire perforé permettent de maintenir cet élément sous forme rétractée afin qu'il puisse être maintenu logé dans le tube guide du dispositif introducteur et de retrouver son diamètre externe de repos après installation in situ.

L'élément tubulaire de forme annulaire (3) du dispositif selon la présente invention, présente au moins un, de préférence plusieurs perforations (10), lesdites perforations étant disposées sur la circonférence dudit élément et en regard de ladite anastomose. La disposition des perforations (10) en regard de l'anastomose permet à la composition de lavement de pouvoir s'écouler facilement en direction de l'anastomose.

L'élément tubulaire du dispositif selon la présente invention comprend de préférence entre 5 et 50, de préférence encore entre 5 et 20 perforations.

Les perforations (10) comprises dans l'élément tubulaire peuvent présenter un diamètre compris entre 0,1 et 5 mm, de préférence entre 0,1 et 2 mm, de préférence encore entre 0,5 et 1 mm. Le nombre et / ou le diamètre des perforations présentes sur l'élément tubulaire sont amenés à varier en fonction du nombre ou du diamètre des perforations, ou encore en fonction débit de distribution de la composition de lavement souhaité.

De préférence, l'élément tubulaire (3) selon la présente invention comprend entre 5 et 10 perforations (10) présentant un diamètre pouvant aller de 0.1 à 1 mm.

Selon un aspect particulier, l'élément tubulaire (3) du dispositif selon la présente invention comprend de multiples perforations (10) régulièrement espacées sur la circonférence de l'élément tubulaire. Cette configuration permet une distribution homogène, de la composition de lavement en amont de l'anastomose et en direction de celle-ci.

Le diamètre externe de l'élément tubulaire (3) avant installation in situ, aussi appelé diamètre externe au repos, peut être compris entre 10 et 70 mm, de préférence entre 20 et 50 mm, de préférence encore entre 20 et 40 mm et le diamètre interne de l'élément tubulaire avant installation in situ, aussi appelé diamètre interne au repos, peut être compris entre 15 et 65 mm, de préférence entre 25 et 45 mm, de préférence entre 35 et 40 mm .

Le diamètre externe de l'élément tubulaire (3) correspond au diamètre le plus grand formé par les parois dudit élément et le diamètre interne correspond au diamètre le plus petit formé par les parois dudit élément.

De préférence, l'élément tubulaire (3) présente un diamètre externe au repos compris entre 20 et 50 mm et un diamètre interne au repos compris entre 15 et 40 mm.

Lorsque l'élément tubulaire (3) du dispositif est fixé sur la partie externe de l'élément d'ancrage (7) du dispositif de protection d'anastomose, le diamètre interne au repos de l'élément tubulaire est similaire au diamètre externe au repos de l'élément d'ancrage du dispositif de protection de l'anastomose. De préférence le diamètre interne au repos de l'élément tubulaire et le diamètre externe au repos de l'élément d'ancrage du dispositif de protection de l'anastomose, sont adaptés en fonction du diamètre du segment intestinal du sujet chez qui ils doivent être insérés.

Le diamètre externe et interne de l'élément tubulaire (3) après installation in situ sont sensiblement inférieurs à celui du diamètre externe et interne au repos dû à la pression de la paroi interne du segment intestinal dans lequel l'élément est installé et aux propriétés d'élasticité des matériaux les composant.

En particulier, le dispositif selon la présente invention est en matériau biocompatible.

Plus précisément, l'élément tubulaire perforé de forme annulaire (3), le tube d'alimentation (4) et les moyens de maintien (6) du dispositif selon la présente invention sont en matériau biocompatible.

Par « matériau biocompatible » ou « biomatériau » on fait référence à un matériau ayant la capacité de ne pas interférer, de ne pas dégrader le milieu biologique dans lequel il est utilisé et ce, même en contact direct ou indirect, bref ou prolongé avec les tissus et fluides internes du corps d'un être humain ou d'un animal. A titre d'exemple de matériaux biocompatibles utilisables dans le cadre de la présente invention, on peut faire référence de façon non exhaustive aux métaux et alliages métalliques tels que le titane, la platine, le nickel, aux polymères d'origine naturelle tels que le collagène, l'agarose, le chitosan, le carraghénane, la cellulose et ses dérivés, le xanthane et l'alginate ou d'origine synthétique tels que les polyesters, les polyoléfines, polyanhydrides, les polymères vinyliques, les polyuréthanes, les polyamides, les polyéther-amides, de préférence les polymères thermosensibles tels que le polyuréthane thermosensible (TPU) ou encore le polyéther-amide thermosensible (TPE-A).

De préférence, le matériau biocompatible utile dans le cadre de la présente invention, est choisi parmi un ou plusieurs matériaux parmi, l'alliage métallique nickel-titane connu sous le nom de nitinol, le polyuréthane thermosensible (TPU), le polyéther-amide thermosensible (TPE-A).

De préférence encore, le matériau biocompatible composant l'élément tubulaire est choisi parmi l'alliage métallique nickel-titane ou le polyéther-amide thermosensible (TPE-A), de préférence encore le polyéther-amide thermosensible (TPE-A).

De préférence encore, le matériau biocompatible composant le tube d'alimentation est le polyuréthane thermosensible (TPU).

De préférence encore, le matériau biocompatible composant les moyens de maintien de l'élément tubulaire est choisi parmi l'alliage métallique nickel-titane ou le polyéther-amide thermosensible (TPE-A), de préférence encore le polyéther-amide thermosensible (TPE-A).

Le matériau biocompatible composant l'élément tubulaire et le tube d'alimentation du dispositif selon la présente invention peuvent être identiques ou différents.

Selon un aspect particulier, les moyens d'injection (5) du dispositif selon la présente invention comprennent une seringue ou une pompe.

Les moyens d'injection (5) compris dans le dispositif selon la présente invention sont aptes à capter, ponctionner, récupérer, aspirer la composition de lavement du dispositif selon l'invention dans le contenant, le récipient, dans lequel elle se trouve.

La composition de lavement est destinée à être insérée dans l'extrémité (4b) du tube d'alimentation (4) s'ouvrant vers des moyens d'injection (5) pour être apportée dans l'élément tubulaire (3) et être distribuée en amont de l'anastomose (2). La composition est choisie en fonction du contexte d'utilisation du dispositif, c'est-à-dire du ou des objectifs du lavement de l'anastomose.

La composition de lavement dans le cadre de la présente invention est une composition dermatologique destinée à et apte à être appliquée sur les muqueuses intestinales.

Dans le cadre de la présente invention, la composition de lavement peut être une solution aqueuse, alcoolique, hydro-alcoolique ou une émulsion pouvant comprendre un ou plusieurs composé(s) actif(s) et un excipient pharmaceutiquement acceptable.

Par exemple, la composition de lavement peut être une émulsion obtenue par dispersion d'une phase grasse dans une phase aqueuse ou vice versa.

Cette composition peut être préparée selon une des méthodes de préparation connues de l'Homme du métier.

En particulier, la composition de lavement du dispositif selon la présente invention est une composition aqueuse, alcoolique ou hydro-alcoolique pouvant comprendre un ou plusieurs composé(s) actif(s) et un excipient pharmaceutiquement acceptable.

Selon un premier mode de réalisation, la composition de lavement du dispositif selon la présente invention est une composition aqueuse, alcoolique ou hydro-alcoolique comprenant un ou plusieurs composé(s) actif(s) et un excipient pharmaceutiquement acceptable. De préférence la composition de lavement est une composition aqueuse.

La composition de lavement peut comprendre plusieurs composés actifs différents ou identiques.

Par « composé actif » dans le cadre de la présente invention, on fait référence à tout composé capable de présenter une activité pharmacologique et / ou un effet physiologique chez le sujet auquel la composition le comprenant est administrée.

Le ou les composés actif(s) compris dans la composition de lavement peuvent être choisis parmi un agent antiseptique, un agent antibactérien, un antibiotique, un agent antiviral, un agent antiparasite, un agent antifongique, un agent anti-allergène, un agent immunosuppresseur, un agent antalgique, un agent anesthésiant, un agent anti-angiogénique, un agent anti-prolifératif, un agent anti-inflammatoire, un agent cicatrisant, un agent probiotique ou un mélange de ces composés.

Plus particulièrement la composition de lavement selon l'invention comprend un ou plusieurs composé(s) actif(s) choisi(s) parmi un agent antiseptique, un agent antibactérien, un agent anti-inflammatoire, un agent antalgique un agent cicatrisant.

Plus particulièrement encore, les composé(s) actif(s) peuvent être choisis parmi un agent antiseptique tel que la chlorhexidine, l'hexamidine, le benzalkonium, l'alcool benzylique, le triclosan, l'iode, un agent antibactérien tel que le butyrate, la demeclocycline, la chlortetracycline, l'oxytétracycline, la tétracycline, le chloramphénicol, l'acide fusidique, la mupirocine, la neomycine, un agent anti inflammatoire tel que le butyrate, les corticoïdes comme l'hydrocortisone la prednisolone, le clobetasol, le desonide, la triamincinolone, le kétoprofène, le diclofénac, le benzydamine, l'ibuprofène, un agent cicatrisant tel quele butyrate, l'acide hyaluronique, le butyrate d'acide hyaluronique le butyrate de sodium,le dexepanthénol, la trolamine, le rétinol ou vitamine A, le cadexomère, le dextranomère, le crilanomère, l'enoxolone, le calcium pantothénate, un agent antalgique tel que les antalgiques non opioïdes comme le diclofénac, l'ibuprofène, le paracétamol, le kétoprofène, les antalgiques opioïdes de palier II comme le tramadol, la codéine, les antalgiques opioïdes de palier III comme le fentanyl, la morphine, l'oxycodone, le sufentanil.

Cette liste de composés actifs est donnée à titre d'exemple, l'Homme du métier saura déterminer les composés actifs pouvant être utilisés dans le cadre de la présente invention.

La composition de lavement peut comprendre entre 0.001 et 10 % de composé(s) actif(s), de préférence entre 0.01 et 8 %, préférentiellement entre 0,1 et 5 %, encore plus préférentiellement entre 0,1 et 3 %, de préférence entre 0,1 et 2 % et de manière encore préférée 2 % en poids par rapport au poids total de la composition.

Selon un aspect préféré, la composition de lavement selon la présente invention comprend 5 % en poids par rapport au poids total de la composition de composé(s) actif(s).

Selon un second mode de réalisation, la composition de lavement du dispositif selon la présente invention est une composition aqueuse, alcoolique ou hydro-alcoolique ne comprenant pas de composé actif, de préférence la composition de lavement est une composition aqueuse telle que du sérum physiologique notamment adapté lorsque l'objectif du lavement est le nettoiement de l'anastomose.

La composition selon la présente invention peut également comprendre des additifs inertes ou pharmacodynamiquement actifs, seuls ou en combinaison, tels que des solvants, des agents mouillants, des agents stabilisants, des agents tensioactifs, des agents conservateurs, des agents régulateurs de pH, des vitamines, des agents émulsifiants, des agents humectants, des agents hydratants, des agents apaisants, des agents pénétrants, des antioxydants, des agents cosmétiquement actifs, des électrolytes, des acides ou bases conventionnels ou des mixtures.

L'Homme du métier veillera à choisir le ou les composé(s) actif(s), les additifs et l'excipient pharmaceutique en fonction du ou des objectif(s) du lavement et de la forme souhaitée de la composition.

Dans le cadre de la présente invention, l'excipient pharmaceutiquement acceptable correspond à tout composé autre que le composé actif et compatible avec les éventuels autres composés présents dans la composition, compatible avec les éléments du dispositif avec lesquels il est en contact, et compatible avec la peau et les muqueuses et en particulier les muqueuses intestinales.

On entend par « sujet » ou « patient » au sens de la présente invention, tout mammifère et plus particulièrement les êtres humains, hommes, femmes, enfants.

La composition de lavement du dispositif selon la présente invention est destinée à être contenue dans un récipient adapté à ladite composition et à partir duquel les moyens d'injections sont aptes à la récupérer.

Le ou les objectif(s) du lavement ont tous pour conséquence directe ou indirecte le traitement et / ou la prévention des complications thérapeutiques liées à la réalisation d'une anastomose et notamment la prévention des fistules anatomiques.

Dans le cadre de la présente invention on entend par « traiter » ou « traitement » une amélioration ou l'inversion d'une maladie ou d'un désordre spécifié ou d'au moins un symptôme discernable ou non discernable. Le terme « traiter » peut aussi désigner la réduction ou le ralentissement de la progression de la maladie ou du désordre, ou de l'apparition des symptômes de cette maladie ou désordre. Par exemple dans le cadre de la présente invention, le terme «traiter » peut correspondre à la réduction ou au ralentissement d'une complication thérapeutique liée à la réalisation d'une anastomose telle qu'une infection bactérienne de l'anastomose, une douleur au niveau de l'anastomose, une difficulté de cicatrisation et / ou à la réduction ou ralentissement d'apparition de fistules anastomotiques.

Dans le cadre de la présente invention on entend par « prévenir » ou «prévention » une réduction du risque d'acquisition d'une maladie ou d'un désordre spécifié, la réduction ou le ralentissement de l'apparition des symptômes de cette maladie. Par exemple dans le cadre de la présente invention, le terme « prévenir » peut correspondre à la réduction du risque d'apparition d'une complication thérapeutique liée à la réalisation d'une anastomose telle qu'une infection bactérienne de l'anastomose, une douleur au niveau de l'anastomose, une difficulté de cicatrisation et / ou à la réduction ou ralentissement d'apparition de fistules anastomotiques.

La présente invention concerne également l'utilisation du dispositif selon le premier objet ou une méthode pour traiter ou prévenir une ou plusieurs complication(s) thérapeutique(s) liée(s) à la réalisation d'une anastomose chez un sujet.

Plus particulièrement, la présente invention concerne l'utilisation du dispositif selon le premier objet ou une méthode pour traiter et / ou prévenir une ou plusieurs complication(s) thérapeutique(s) liée(s) à la réalisation d'une anastomose chez un sujet, dans laquelle le dispositif, en particulier l'élément tubulaire dudit dispositif, est installé in situ en amont de ladite anastomose, et la composition de lavement est insérée par des moyens d'injection dans l'extrémité du tube d'alimentation du dispositif s'ouvrant vers lesdits moyens d'injection pour être distribuée en amont de l'anastomose par l'intermédiaire dudit élément tubulaire. La composition de lavement et la quantité de ladite composition sont déterminées en fonction du ou des complication(s) thérapeutique(s) ciblées.

La présente invention concerne également un dispositif selon le premier objet pour son utilisation pour traiter ou prévenir une ou plusieurs complication(s) thérapeutique(s) liée(s) à la réalisation d'une anastomose chez un sujet.

Plus particulièrement, la présente invention concerne un dispositif selon le premier objet pour son utilisation pour traiter et / ou prévenir une ou plusieurs complication(s) thérapeutique(s) liée(s) à la réalisation d'une anastomose chez un sujet, dans laquelle le dispositif, en particulier l'élément tubulaire dudit dispositif, est installé in situ en amont de ladite anastomose, et la composition de lavement est insérée par des moyens d'injection dans l'extrémité du tube d'alimentation du dispositif s'ouvrant vers lesdits moyens d'injection pour être distribuée en amont de l'anastomose par l'intermédiaire dudit élément tubulaire. La composition de lavement et la quantité de ladite composition sont déterminées en fonction du ou des complication(s) thérapeutique(s) ciblées.

Dans le cadre des utilisations et méthodes susmentionnées, les moyens de maintien du dispositif selon la présente invention sont fixés sur l'élément d'ancrage, notamment le stent, d'un dispositif de protection d'anastomose avant son installation in situ. En d'autres termes, le dispositif selon la présente invention est couplé au dispositif de protection d'anastomose avant son installation in situ.

De préférence, le dispositif selon la présente invention est installé en amont de l'anastomose après la réalisation de celle-ci via l'orifice anal par un dispositif introducteur tel que décrit en relation avec le premier objet de la présente invention. Plus précisément, le dispositif introducteur peut être constitué d'un tube guide semi rigide, de type cathéter muni à une de ses extrémités d'une poignée et dont le diamètre interne et la longueur permettent d'y maintenir logé l'élément d'ancrage du dispositif de protection d'anastomose donc de préférence le stent et l'élément tubulaire du dispositif selon la présente invention, sous leur forme rétractée et la gaine du dispositif de protection d'anastomose et le tube d'alimentation du dispositif selon la présente invention, déployés longitudinalement.

De préférence, le dispositif selon la présente invention est installé en amont de l'anastomose de suite après la réalisation de l'anastomose c'est-à-dire à l'issue de l'intervention chirurgicale.

Dans le contexte des utilisations et méthodes susmentionnées, le dispositif selon la présente invention peut être utilisé seul ou combiné à l'administration simultanée ou séquentielle d'autres composés médicamenteux tels que des agents antibactériens, des agents antalgiques, des agents cicatrisants lesdits composés pouvant être administrés par voie entérale telle que par voie buccale ou anale, par voie parentérale telle que par voie intraveineuse ou autres.

Dans le cadre des utilisations ou méthodes susmentionnées, le dispositif et en particulier l'élément tubulaire dudit dispositif est installé en amont de ladite anastomose à une distance comprise entre 20 et 5 cm, de préférence entre 15 et 10 cm de la zone où l'anastomose doit être réalisée.

Dans le cadre des utilisations ou méthodes susmentionnées, la composition de lavement est distribuée de façon répétée en amont de l'anastomose une à plusieurs fois par jour, de préférence au moins 1 fois par jour, sur une période adaptée à l'objectif du lavement.

La période adaptée au lavement peut être comprise entre 1 jour à 3 semaines, de préférence de 1 jour à 15 jours, de préférence encore 9 jours. De préférence dans le cadre des utilisations ou méthodes susmentionnées, la composition de lavement est distribuée en amont de l'anastomose une fois par jour pendant 9 jours à compter du deuxième jour suivant la réalisation de l'anastomose.

Dans le cadre des utilisations ou méthodes susmentionnées, différentes compositions de lavement peut être distribuées en amont de l'anastomose, de façon simultanée ou à différents moments avec un délai entre chaque mise en oeuvre.

L'Homme du métier saura déterminer les paramètres de traitement et / ou prévention à mettre en oeuvre tels que la quantité de composition de lavement insérer, la fréquence d'administration, le(s) composé(s) actif(s) présent(s) dans la composition, le dosage de chaque composé actif et la forme de composition.

La présente invention concerne également un procédé d'installation en amont d'une anastomose dans lequel :
- le dispositif de lavement (1) selon le premier objet est couplé au dispositif de protection d'anastomose, en particulier l'élément tubulaire (3) du dispositif selon l'invention est fixé à l'élément d'ancrage (7) du dispositif de protection d'anastomose
- l'élément tubulaire (3) du dispositif selon la présente invention et l'élément d'ancrage (7) du dispositif de protection d'anastomose sont logés sous forme rétractée au sein de l'extrémité distale du tube guide semi rigide (20) d'un dispositif introducteur constitué dudit tube muni d'une poignée à l'une de ses extrémités
- l'extrémité distale du tube guide du dispositif introducteur est introduit depuis l'orifice anal jusqu'en amont de l'anastomose par des moyens adaptés
- l'élément d'ancrage et l'élément tubulaire sont libérés du tube guide par des moyens adaptés.

Les moyens pour acheminer l'élément d'ancrage et l'élément tubulaire depuis l'orifice anal jusqu'en amont de l'anastomose peuvent être un pousseur s'étendant depuis la poignée reliée à l'extrémité du tube guide et comprenant une tige de pousseur et une butée de pousseur à l'extrémité distale de la tige de pousseur. La butée du pousseur étant apte à libérer l'élément d'ancrage et l'élément tubulaire en les poussant à l'extérieur de l'extrémité distale du tube guide pour permettre leur extension radiale et l'ancrage de l'élément d'ancrage, notamment du stent, contre la paroi de l'intestin.

De préférence, le procédé d'installation est mis en oeuvre de suite après la réalisation de l'anastomose c'est-à-dire à l'issue de l'intervention chirurgicale.

Le dispositif de lavement selon la présente invention est retiré à la fin de la période de lavement choisie, par technique d'endoscopie à l'aide d'une pince endoscopique. Plus précisément, l'élément d'ancrage c'est-à-dire le stent (7) du dispositif de protection de l'anastomose auquel est couplé l'élément tubulaire (3) du dispositif selon l'invention est dans un premier temps détaché de la paroi intestinale par l'extrémité amont du stent puis est tiré par l'extrémité avale du stent hors du corps du sujet par l'anus.

La présente invention concerne également un kit comprenant le dispositif (1) selon le premier objet de l'invention comprenant des moyens d'injection (5) et un dispositif de protection d'anastomose comprenant une gaine externe souple (9) liée à un stent (7) situé en aval de la gaine, ledit stent étant destiné à être ancré en amont de l'anastomose. De préférence, le kit comprend également une composition de lavement.

De préférence, le kit selon l'invention comprend le dispositif selon le premier objet de l'invention comprenant un élément tubulaire perforé de forme annulaire apte à distribuer une composition de lavement en amont de l'anastomose, un tube d'alimentation dont une extrémité débouche dans l'élément tubulaire et une extrémité s'ouvre vers des moyens d'injection destiné à apporter la composition dans ledit élément tubulaire, des moyens de maintien de l'élément tubulaire en amont de l'anastomose, des moyens d'injection d'une composition de lavement et un dispositif de protection d'anastomose comprenant une gaine externe souple liée à un stent situé en aval de la gaine, ledit stent étant destiné à être ancré en amont de l'anastomose.

Les moyens d'injection (5) peuvent être une seringue ou une pompe, de préférence une seringue.

En particulier, le kit selon la présente invention comprend également un récipient comprenant une composition de lavement prête à être insérée par l'extrémité libre du tube du dispositif selon le premier objet.

Selon un aspect préféré, le kit selon la présente invention comprend le dispositif selon le premier objet de l'invention couplé au dispositif de protection de l'anastomose, c'est-à-dire dans lequel l'élément tubulaire (3) du dispositif selon la présente invention est fixé par des moyens de maintien (5) sur l'élément d'ancrage (7) du dispositif de protection d'anastomose.

De préférence, le kit selon la présente invention comprend également un dispositif introducteur apte à insérer le dispositif selon la présente invention couplé au dispositif protecteur d'anastomose, en amont de l'anastomose. Le dispositif introducteur peut être constitué d'un tube guide semi rigide (20), de type cathéter muni à une de ses extrémités d'une poignée et dont le diamètre interne et la longueur permettent d'y maintenir logé l'élément d'ancrage du dispositif de protection d'anastomose donc de préférence le stent et l'élément tubulaire du dispositif selon la présente invention, sous leur forme rétractée et la gaine du dispositif de protection d'anastomose et le tube d'alimentation du dispositif selon la présente invention, déployés longitudinalement.

Le kit selon la présente invention comprend de préférence l'élément tubulaire (3) du dispositif selon la présente invention et l'élément d'ancrage (7) du dispositif de protection d'anastomose sous forme rétractée au sein du tube guide (20) du dispositif introducteur.

L'invention sera davantage illustrée par les figures suivantes. Cependant, ces figures ne doivent en aucun cas être interprétées comme limitant la portée de l'invention.

## Revendications

1. Kit comprenant un dispositif (1) pour le lavement d'une anastomose intestinale (2) in situ et un dispositif de protection d'anastomose comprenant une gaine externe souple (9) liée à un stent (7) situé en aval de la gaine, ledit stent étant destiné à être ancré en amont de l'anastomose, le dispositif (1) pour le lavement comprenant un élément tubulaire perforé de forme annulaire (3), apte à distribuer une composition de lavement en amont de l'anastomose, un tube d'alimentation (4) dont une extrémité (4a) débouche dans l'élément tubulaire et une extrémité (4b) s'ouvre vers des moyens d'injection (5) destinés à apporter la composition dans ledit élément tubulaire, des moyens de maintien (6) de l'élément tubulaire en amont de l'anastomose.

2. Kit selon la revendication 1 dans lequel les moyens de maintien (6) du dispositif (1) pour le lavement sont disposés sur l'élément tubulaire.

3. Kit selon l'une des revendications 1 ou 2 dans lequel les moyens de maintien (6) du dispositif (1) pour le lavement sont fixés sur le stent (7) du dispositif de protection d'anastomose.

4. Kit selon l'une des revendications précédentes dans lequel le tube d'alimentation (4) du dispositif (1) pour le lavement présente un diamètre interne compris entre 1 et 8 mm.

5. Kit selon l'une des revendications précédentes dans lequel l'élément tubulaire (3) du dispositif (1) pour le lavement comprend de multiples perforations (10) régulièrement espacées sur la circonférence de l'élément tubulaire.

6. Kit selon l'une des revendications précédentes dans lequel le dispositif (1) pour le lavement est en matériau biocompatible.

7. Kit selon l'une des revendications précédentes dans lequel les moyens d'injection (5) du dispositif (1) pour le lavement comprennent une seringue ou une pompe.

8. Kit selon l'une des revendications précédentes, comprenant une composition de lavement.

9. Kit selon l'une des revendications précédentes dans lequel l'élément tubulaire (3) du dispositif (1) pour le lavement est fixé par des moyens de maintien (6) sur le stent (7).

## Patentansprüche

1. Kit aufweisend eine Spülvorrichtung (1) zum Spülen einer Darmanastomose (2) in situ und eine Anastomose-Schutzvorrichtung, die eine weiche Außenhülle (9) aufweist, die mit einem stromab der Hülle angeordneten Stent (7) verbunden ist, wobei der Stent vorgesehen ist, stromauf der Anastomose verankert zu werden, wobei die Spülvorrichtung (1) aufweist: ein perforiertes ringförmiges Röhrenelement (3), das fähig ist, eine Spülzusammensetzung stromauf der Anastomose zu verteilen, einen Zuleitungsschlauch (4), ein Ende (4a) dessen in das Röhrenelement mündet und ein Ende (4b) sich zu einer Injektionseinrichtung (5) öffnet, die vorgesehen ist, die Zusammensetzung in das Röhrenelement zu bringen, eine Halteeinrichtung (6) zum Halten des Röhrenelements stromauf der Anastomose.

2. Kit nach Anspruch 1, wobei die Halteeinrichtung (6) der Spülvorrichtung (1) an dem Röhrenelement angeordnet ist.

3. Kit nach einem der Ansprüche 1 oder 2, wobei die Halteeinrichtung (6) der Spülvorrichtung (1) an dem Stent (7) der Anastomose-Schutzvorrichtung angebracht sind.

4. Kit nach einem der vorstehenden Ansprüche, wobei der Zuleitungsschlauch (4) der Spülvorrichtung (1) einen Innendurchmesser zwischen 1 und 8mm hat.

5. Kit nach einem der vorstehenden Ansprüche, wobei das Röhrenelement (3) der Spülvorrichtung (1) mehrere Perforationen (10) aufweist, die am Umfang des Röhrenelements regelmäßig beabstandet sind.

6. Kit nach einem der vorstehenden Ansprüche, wobei die Spülvorrichtung (1) aus einem biokompatiblen Material ist.

7. Kit nach einem der vorstehenden Ansprüche, wobei die Injektionseinrichtung (5) der Spülvorrichtung (1) eine Spritze oder eine Pumpe aufweist.

8. Kit nach einem der vorstehenden Ansprüche, aufweisend eine Spülzusammensetzung.

9. Kit nach einem der vorstehenden Ansprüche, wobei das Röhrenelement (3) der Spülvorrichtung (1) mittels einer Halteeinrichtung (6) an dem Stent (7) befestigt ist.

## Claims

1. Kit comprising a device (1) for intestinal anastomosis enema (2) in situ, and an anastomosis protection device comprising a flexible external sheath (9) connected to a stent (7) located downstream of the sheath, said stent being designed to be anchored upstream of the anastomosis, the enema device (1) comprising a perforated, tubular element of annular shape (3) that can distribute an enema composition, upstream of the anastomosis, a supply tube (4) one end of which (4a) emerges in the tubular element and one end of which (4b) is open to injection means (5) designed to deliver the composition into said tubular element, holding means (6) for the tubular element upstream of the anastomosis.

2. Kit according to claim 1 wherein the holding means (6) of the enema device (1) are positioned on the tubular element.

3. Kit according to one of claims 1 or 2 wherein the holding means (6) of the enema device (1) are attached on the stent (7) of the anastomosis protection device.

4. kit according to one of the preceding claims wherein the supply tube (4) of the enema device (1) has an inner diameter comprised between 1 and 8 mm.

5. Kit according to one of the preceding claims wherein the tubular element (3) of the enema device (1) comprises multiple perforations (10) regularly spaced on the circumference of the tubular element.

6. Kit according to one of the preceding claims, wherein the enema device (1) is of biocompatible material.

7. Kit according to one of the preceding claims wherein the injection means (5) of the enema device (1) comprise a syringe or a pump.

8. Kit according to one of the preceding claims comprising an enema composition.

9. Kit according to one of the preceding claims wherein the tubular element (3) of the enema device (1) is attached by holding means (6) on the stent (7).
